# EUROPEAN PATENT SPECIFICATION

(11) **EP 1 734 131 B1**
(45) Date of publication and mention of the grant of the patent: **15.08.2012**
(21) Application number: 06460014.1
(22) Date of filing: 14.06.2006
(51) Int. Cl.: A23K 1/16, A61K 38/16

(54) **A process of manufacturing a lectin preparation, the lectin preparation and methods of administration of the preparation to mammals**
Verfahren zur Herstellung eines Lektinpräparats, das Lektinpräparat und dessen Applikationsmethode bei Säugetieren
Procédé d'obtention d'une préparation de lectine, ladite préparation de lectine, et méthodes d'administration de ladite préparation à des mammifères

(30) Priority: 16.06.2005 PL 37574605
(43) Date of publication of application: 20.12.2006
(73) Proprietor: Biolek SP. z o.o., 01-231 Warszawa (PL)
(72) Inventor: Laubitz, Daniel, 01-926 Warszawa (PL); Michalowski, Pawel, 05-126 Nieporet (PL); Valverde Piedra, Jose L., 21-003 Ciecierzyn (PL); Pierzynowski, Stefan G., 240 32 Flyinge (SE); Weström, Björn R., 262 52 Ängelholm (SE); Wolinski, Jaroslaw, 06-100 Pultusk (PL); Zabielski, Romuald, 02-997 Warszawa (PL)
(74) Representative: Witek, Rafal

(56) References cited:
- WO-A-00/27876
- WO-A-97/49420
- DE-C1- 3 524 426
- PL-A1- 330 777
- RU-C1- 2 115 427
- US-A- 5 053 386
- DATABASE WPI Section Ch, Week 197149 Derwent Publications Ltd., London, GB; Class B04, AN 1971-77779S XP002399312 & SU 291 710 A (BERGMANIS LA KARMANSKII I) 1971
- DATABASE WPI Section Ch, Week 199735 Derwent Publications Ltd., London, GB; Class B04, AN 1997-375572 XP002399313 & HU 210 860 A (SEMMELWEIS ORVOSTUDOMANYI EGYETEM) 28 December 1995 (1995-12-28)
- DATABASE WPI Week 199318 Derwent Publications Ltd., London, GB; AN 1993-150103 XP002399314 & SU 1 732 998 A1 (POLT AGRIC INST) 15 May 1992 (1992-05-15)
- DATABASE WPI Week 199738 Derwent Publications Ltd., London, GB; AN 1997-413821 XP002399315 & SU 1 580 614 A1 (UNIV SARAT) 10 March 1997 (1997-03-10)
- PUSZTAI A ET AL: "Isolectins of Phaseolus vulgaris. A comprehensive study of fractionation", BBA - PROTEIN STRUCTURE, ELSEVIER SCIENCE BV, AMSTERDAM, NL, vol. 365, no. 1, 13 September 1974 (1974-09-13), pages 57-71, XP024555492, ISSN: 0005-2795, DOI: DOI:10.1016/0005-2795(74)90250-5 [retrieved on 1974-09-13]
- PUSZTAI A ET AL: "GLYCO PROTEIN II THE ISOLATION AND CHARACTERIZATION OF A MAJOR ANTIGENIC AND NON-HEMAGGLUTINATING GLYCOPROTEIN FROM PHASEOLUS VULGARIS", BIOCHIMICA ET BIOPHYSICA ACTA, ELSEVIER, NL, vol. 207, no. 3, 15 March 1970 (1970-03-15), pages 413-431, XP008102670, ISSN: 0006-3002
- RADBERG K ET AL: "Enteral exposure to crude red kidney bean lectin induces maturation of the gut in suckling pigs", JOURNAL OF ANIMAL SCIENCE, AMERICAN SOCIETY OF ANIMAL SCIENCE, US, vol. 79, 1 January 2001 (2001-01-01), pages 2669-2678, XP002962548, ISSN: 0021-8812
- EVILEVITCH L ET AL: "Three-day enteral exposure to a red kidney bean lectin preparation enhances the pancreatic response to CCK stimulation in suckling pigs", BIOLOGY OF THE NEONATE, KARGER, BASEL, CH, vol. 87, no. 1, 1 January 2005 (2005-01-01), pages 20-25, XP008102555, ISSN: 0006-3126, DOI: DOI:10.1159/000080896 [retrieved on 2004-09-16]
- CARVALHO MARIA REGINA B ET AL: "Relative importance of phytohemagglutinin (lectin) and trypsin-chymotrypsin inhibitor on bean (Phaseolus vulgaris L) protein absorption and utilization by the rat", JOURNAL OF NUTRITIONAL SCIENCE AND VITAMINOLOGY, UNIVERSITY OF TOKYO PRESS, TOKYO, JP, vol. 44, no. 5, 1 October 1998 (1998-10-01), pages 685-696, XP008102741, ISSN: 0301-4800
- A. Thomsson ET AL: "Effects of crude red kidney bean lectin (phytohemagglutinin) exposure on performance, health, feeding behavior, and gut maturation of pigs at weaning", Journal of Animal Science, vol. 85, no. 2, 1 February 2007 (2007-02-01), pages 477-485, XP55011558, ISSN: 0021-8812, DOI: 10.2527/jas.2006-250
- WANG H X ET AL: "ISOLATION AND CHARACTERIZATION OF TWO DISTINCT LECTINS WITH ANTIPROLIFERATIVE ACTIVITY FROM THE CULTURED MYCELIUM OF THE EDIBLEMUSHROOM TRICHOLOMA MONGOLICUM", INTERNATIONAL JOURNAL OF PEPTIDE AND PROTEIN RESEARCH, MUNKSGAARD, COPENHAGEN, DK, vol. 46, no. 6, 1 December 1995 (1995-12-01), pages 508-513, XP000538874, ISSN: 0367-8377
- ALLEN A K ET AL: "Vicia faba lectin", METHODS IN ENZYMOLOGY; [METHODS IN ENZYMOLOGY], ACADEMIC PRESS INC, SAN DIEGO, CA, US, vol. 50, 1 January 1978 (1978-01-01), pages 335-339, XP008122224, ISSN: 0076-6879
- Evaluation report http://irmm.jrc.ec.europa.eu/SiteCollectio nDocuments/FinRep-FAD-2010-0079.pdf
- VALVERDE ET AL.: "Suilectin (R) - nowy preparat w profilaktyce odsadzania prosiat", VETERINARY MEDICINE - SCIENCE & PRACTICE, vol. 61, September 2005 (2005-09), page 29,

## Description

An object of the invention is the process of manufacturing of a lectin preparation, and the lectin preparation, especially to increase the weight gain in weaned piglets.

The way of administration of the preparations is *per os* (oral).

The preparation preventing the diarrhoea in piglets, especially in the period of weaning from their mothers, is known from Polish patent description no. P.332344, and contains the zinc oxide and sulphaguanidine mixed in suitable quantities with the carrier. Such a preparation is prepared by choosing the appropriate quantity of these compounds and their exact mixing to achieve the homogenous substance. After positive analysis the preparation is packed in the tight containers and distributed.

The method to decrease the rate of diarrhoea after weaning in young mammals, especially in farm animals, and diarrhoea in children, known from Polish description application of the invention no. P.330777, is characterized by inducing the precocious maturation of the intestine and the process of its adaptation to food by the acceleration of epithelium cells exchange in the digestive system on the way of giving orally or into the stomach the additional lectins, profitably phytohaemagglutinin, isolated from the kidney bean in a dose from 10 to 500 mg/kg of body weight before the weaning to meet the requirements of proper digestion and food utilization.

Pusztai A. And Watt W.B. (Biochim.Biophys.Acta, vol. 365, 1974, p.57-71) discloses dried lectin preparations obtained from kidney beans, and refers to an earlier publication of Pusztai A. And Watt W.B. (Biochim.Biophys.Acta, vol. 207, 1970, p.413-431) which provides the definition of the haemagglutinin unit and an assay for determining it, to be used according to the invention.

Radberg K. et al. (J.Anim.Sci., vol. 79, 2001, p. 2669-2678) and Evilevitch L. et al. (Biol.Neonate, vol. 87, 2004-09-16, p. 20-26) disclosed that thusly obtained lectin preparations can be administrated to suckling piglets. In the described experiments, 100 miligrams of lectin per kilogram of body weight were administrated to the animals.

Carvalho M.R.B. et al. (J.Nutri.Sci.Vitaminol., vol. 44, 1998, p.685-696) disclosed another process for purifying lectin from beans (Phaseolus vulgaris L), and to make a preparation suitable for administration to animals. The lectins are extracted from bean flour (i.e. milled beans) with an acidic salt solution followed by fractionation via dialysis and centrifugation into an albumin fraction and globulin fraction, and finally freeze-drying (i.e. clotting) of the fractions.

### Subject of the invention

The first subject of the invention is the use of lectin in the manufacture of a preparation for increase in the weight gain in the weaned piglet, where said lectin preparation is in a dry or liquid form and it is orally administered to said piglet one time between the 10th and 15th day of life as a unit dosage comprising 320 haemagglutinin activity units (HU) for animals weighting below 2 kg and 640 HU for animals weighting 2-5 kg, wherein one unit of haemagglutinin activity (HU) is the quantity of material (in mg) which causes 50% agglutination of 1 ml of a 2% erythrocyte suspension at temperature of 25°C, and wherein said lectin preparation:
- is apportioned in containers with a marked level to which water can be introduced to obtain the suspension with haemagglutinin activity either 320HU or 640 HU/ml or
- is in a dry form of tablets, containing, dried lectin powder and talc and/or monosaccharides, preferably lactose and/or crystalline cellulose and/or lubricants, preferably magnesium stearate, and said tablets are coated with the film or sugar to facilitate swallowing and the haemagglutinin activity of each tablet or coated tablet or dragee is either 320HU or 640 HU or
- is in a dry form of gelatinate or cellulose capsules, containing the dried lectin powder and the filler, wherein the haemagglutinin activity of each capsule is either 320HU or 640 HU.

The second subject of the invention is a lectin preparation for use in increasing the weight gain of weaned piglets, where said lectin preparation is orally administered to said piglet one time between the 10th and 15th day of life in dose comprising 320 haemagglutinin activity units (HU) for animals weighing below 2 kg and 640 HU for animals weighting 2-5 kg, wherein one unit of haemagglutinin activity (HU) is the quantity of material (in mg) which causes 50% agglutination of 1 ml of a 2% erythrocyte suspension at temperature of 25°C.

### Detailed description of the embodiments

The following description comprises additional information facilitating the embodiments of the invention defined above. These are given only for illustrative purposes and should not be taken as the scope of the claimed invention.

The process of manufacturing of lectin preparation according to the invention distinguishes itself that the preparation is obtained on the one-step process of acid extraction of minced seeds of leguminous plants in the presence of water soluble natural antioxidants, and that process of milling of the seeds of leguminous plants is performed in the temperature below 80°C and after the separating of the solid pieces the extract undergoes concentration process, and thereafter the analysis is performed to assess the biological activity, and after the standardization the product is container filled or the raw extract is spray-dried and after standardization is directed to boxing.

It is profitable if haemagglutinic activity HU (haemagglutinic unit) is marked as biological activity, defined as a quantity of material expressed in mg/ml in the last dilution causing 50% agglutination of 2% erythrocytes suspension in temperature +25° C, and the seeds of leguminous plants are the seeds of kidney bean.

Besides, it is profitably if the acid extraction of minced seeds of leguminous plants is conducted in the presence of natural antioxidants, and the process of milling of seeds of leguminous plants is performed in the temperature 30-40° C, and extract concentration and separation is done using the technology of reversed osmosis, the concentrated extract is standardized, the preservatives and stabilizers are added for liquid form of the preparation.

According to the invention to receive a dry form of the preparation the concentrated extract is submitted to drying. Dried extract is analyzed for haemagglutinic activity HU, and thereafter standardized, and the conservatives and stabilizers are added. It is profitable if the drying process of concentrated extract is conducted with spray-drying process.

The lectin preparation for the mammals distinguishes itself that as a dry form for the suspension *ex tempore* contains a dry lectin powder obtained by the single-step acid extraction of minced seeds of leguminous plants in the presence of water soluble natural antioxidants, and successively the concentration and separation with reversed osmosis technique and spray-drying technique with defined haemagglutinic activity HU in distinguished volume (ml) of the suspension, containing suspension stabilizer and/or aromatic substances and/or palatability substances.

It is profitable if the lectin preparation for preparing the *ex tempore* suspension is bottled or boxed with a marked level to which water can be added to obtain the suspension with a haemagglutinic activity of 320-640 HU/ml.

First other lectin preparation for the mammals distinguishes itself that as a dry form are tablets containing dried lectin powder, and talc, and/or monosaccharides, especially lactose, and/or crystalline cellulose, and/or lubricants, profitably magnesium stearate.

It is profitable if tablets of the first different lectin preparation are coated with the film or sugar coating to facilitate swallowing and haemagglutinic activity of each tablet or coated tablet or dragee is 320-640 HU.

The second other lectin preparation for the mammalian distinguishes itself that as a dry form are gelatine or cellulose capsules form containing dried lectin powder and the auxiliary substances, and haemagglutinic activity of each capsule is 320-640 HU.

The third other lectin preparation for the mammalian distinguishes itself that as a liquid form of suspension are dried lectin powder or concentrated lectin extract, preservative, and/or stabilizer, and/or palatability substances, profitably monosaccharides, and/or aromatic substances and water or aqueous salt solutions, and haemagglutinic activity of the suspension is 320-640 HU/ml.

The fourth other lectin preparation for the mammalian distinguishes itself that as a liquid gel form are dried lectin powder or concentrated lectin extract, preservative, and/or stabilizer, and/or palatability substances, profitably monosaccharides, and/or aromatic substances, gelatine substances, and water, and haemagglutinic activity of the gel is 320-640 HU/ml.

It is profitable if the preparation in a liquid form is confectioned in tube-syringes with or without the possibility to dosage setting or confectioned in the containers adapted to automatic batchers with or without the possibility of dosage setting.

The lectin preparation for the mammalian is administered as a dry or liquid form *per os* (orally) to clinically healthy mammals one time between the 10^{th} and 15^{th} day of their life, especially to the piglets, with an activity of 320 haemagglutinic units for animals weighting below 2 kg, and 640 haemagglutinic units for animals weighting 2-5 kg.

In reasonable cases the lectin preparation is administered to the mammals by tubing to the oesophagus and/or stomach directly.

According to the invention the lectin preparation given *per os* (orally) acts superficially and profitably on digestive system causing the increase of the weight gain and better utilization of the feed.

According to the invention the lectin preparation is subjected with the haemagglutinic test which is the only method to qualify its biological activity HU, not its quantity. One-stage process of acid extraction of minced bean seeds allows to obtain one solid waste which after the separation of aqueous extract is a rich source of proteins devoid of anti-nutritional substances and after desiccation and standardization can be used as protein-rich additive in feed production. The way of production of liquid and dry forms guarantees high stability of the lectin preparation and gives the possibility of easy administration of the preparation.

The object of the invention is precisely explained below as the example of its production.

The lectin preparation, trade name Suilectin, is manufactured in a one-step process of acid extraction of minced beans seeds in the presence of water soluble natural antioxidants in the temperature below +30°C, and after the separation of the solid phase the extract undergoes the concentration process and separation by reversed osmosis technique. Concentrated extract is analyzed to calculate the biological activity. To unify and simplify the evaluation of the content of biologically active lectin in the preparation administered to the animals, a haemagglutinic activity HU is measured both in the finished preparation and in the indirect stages of the manufacturing of the trade preparation Suilectin.

One unit of the haemagglutinic activity HU is defined as an amount of the material in mg/ml in the last dilution causing 50% agglutination of 2% erythrocyte suspension in temperature +25° C according to the method of Pusztai and Watt, with the modification in 1974.

The concentrated extract according to a final form of lectin preparation is processed as follows, to obtain liquid forms it undergoes the standardization process and conservatives and stabilizers are added or to obtain dry forms the drying process is performed.

The drying process of concentrated extract is performed using spray-drying technique, thereafter a dry powder is analyzed to calculate haemagglutinic activity HU and standardized.

The lectin preparation for the mammals can be obtained in dry and liquid form. The dry forms of the preparation include the powder for receiving the suspension *ex tempore,* tablets and capsules.

The dry form of the preparation to prepare the suspension *ex tempore* is the dried powder obtained on the way of the one-step acid extraction, concentration and drying the extract of minced seeds of leguminous plants with definite haemagglutinic activity HU in 1 ml of the suspension, containing stabilizer and/or aromatic substances and/or palatability substances. The preparation is confectioned in the containers with marked level to which water is introduced to obtain the suspension with haemagglutinic activity of 320-640 HU/ml.

The dry form of tablets contains dried lectin powder and talc, and/or monosaccharides, profitably lactose, and/or crystalline cellulose, and/or lubricants, profitably magnesium stearate. The tablets are coated with film or sugar coat to facilitate swallowing and haemagglutinic activity of each tablet or coated tablet or dragee is 320-640 HU.

The dry form of gelatine or cellulose capsules contains dried lectin powder and the auxiliary substances and has hemaglutinic activity 320-640 HU/capsule.

The liquid form of suspension contains dried lectin powder or concentrated lectin extract, the preservative, and/or stabilizer and/or palatability substances, profitably monosaccharides, and/or aromatic substances, and/or water, and haemagglutinic activity of the suspension is 320-640 HU/ml.

The liquid form of gel contains dried lectin powder or concentrated lectin extract, the preservative, and/or the stabilizer, and/or gustatory substances, profitably monosaccharides and/or aromatic substances, gelatine substances, and/or water and haemagglutinic activity of 320-640 HU/ml.

The preparation in a liquid form is confectioned in tube-syringes with or without the possibility of dosage setting or in the containers adapted for automatic batchers with or without the possibility of dosage setting.

The example effects of lectin preparation Suilectin in pig farms.

Four pig farms from Provinces Lubelskie, Mazowieckie and Podlaskie participated in the study. A total number of 298 piglets of both sexes in the postnatal age of 10-63 days were included into the study, and the whole litters consisting of 9-14 animals were analyzed. In each farm the piglets were divided randomly into a control and experimental group. At 10^{th}-14^{th} day of life the clinically healthy piglets from the experimental group received Suilectin in a single oral (*per os*) dose of 320 HU for piglets weighting below 2 kg and 640 HU for piglets weighting 2-5 kg. The piglets of the both control and experimental group were fed with mother's milk until weaning. In farms 3 and 4, presented in Table 1, the piglets additionally received the prestarter feed since the 14^{th} day of life. Piglets were weaned in 28^{th} day of their life, and then fed with full-portion weaned feed according to standard feeding procedure in the farm. In farms 1 and 3 in Table 1, the feed was prepared individually with the use of own materials and vitamin-mineral mixture Lutamiks (BASF, Kutno, Poland), which did not contain zinc oxide, antibiotics and other growth promoters. In farms 2 and 4 in Table 1, piglets were fed with the full-portion feedstuff suitable for their age containing antibiotic growth promoters and zinc oxide.

The piglets body weight was controlled in a day of administration of Suilectin, on the day of weaning, 3 and 7 days after weaning, and thereafter in a week intervals till 49^{th}, 56^{th} or 63^{rd} day of life. Daily feed consumption was controlled in 2 farms. After completing the study the piglets were further routinely fed and kept to achieve the slaughter body mass.

The results of the study indicate the profitable effect of lectin preparation administered one time *per os* (orally) in a form of capsules or suspension in 10-14^{th} day of life to clinically healthy piglets. In 49^{th} day of life piglets in the experimental group had significantly higher body weight in comparison with piglets from control group as shown in Table 1.

The dynamics of the body weight gain measured between the day of administration of lectin preparation and weaning was higher in the group of piglets receiving lectin preparation as compared to controls. The feed conversion ratio indicated better utilization of the feedstuff by pigs in the experimental groups than in controls.

**Table 1. The average body weight and daily weight gains in control group and group treated with Suilectin. Values represent averaged results ± SE from 2 larger farms n° 1 and 2, and 2 smaller farms n° 3 and 4 in which the feed containing antibiotic growth promoters + ASW or not -ASW was applied.**

| | Body weight in kg | | | Daily increases in body weight in g | |
|---|---|---|---|---|---|
| Farm | Age | Suilectin | Control | Suilectin | Control |
| 1 | 10-14 | 4.1 ±0.1 | 4.2 ±0.1 | | |
| -ASW | 28 | 8.0 ±0.2 | 8.1 ±0.3 | 230 ±7.3 | 225 ±8.8 |
| n=109 | 63 | 20.9 ±0.3* | 19.4 ±0.7 | 367 ±10.0** | 324 ±15.0 |
| 2 | 10-14 | 4.1±0.1 | 4.0±0.1 | | |
| +ASW | 28 | 7.5 ±0.2* | 7.0 ±0.2 | 198 ±7.2 | 177 ±6.0 |
| n=108 | 49 | 13.6±0.4*** | 10.8 ±0.2 | 298 ±14.2 | 182 ±7.2 |
| 3 | 10-14 | 4.2 ±0.2** | 3.6 ±0.2 | | |
| -ASW | 28 | 9.8 ±0.4** | 8.3 ±0.3 | 325±15.1** | 278 ±12.0 |
| n= 38 | 56 | 18.4 ±0.7 | 16.8 ±0.8 | 309 ±22.5 | 301 ±24.8 |
| 4 | 10-14 | 3.4±0.2 | 3.6 ±0.2 | | |
| +ASW | 28 | 6.4 ±0.4 | 6.3 ±0.3 | 177±16.8 | 158 ±11.4 |
| n= 43 | 56 | 18.1 ±0.1 | 17.1 ±0.9 | 334 ± 17.0 | 261 ±37.6 |

| | | | | | |
|---|---|---|---|---|---|
| Statistical differences were evaluated with t-student test. * p < 0.05. ** p < 0.01. *** p < 0.001. | | | | | |

## Claims

1. Use of lectin in the manufacture of a preparation for increase in the weight gain in the weaned piglet, where said lectin preparation is in a dry or liquid form and it is orally administered to said piglet one time between the 10^{th} and 15^{th} day of their life as a unit dosage comprising 320 haemagglutinic activity units (HU) for animals weighting below 2 kg and 640 HU for animals weighting 2-5 kg, wherein one unit of haemagglutinic activity (HU) is the quantity of material (in mg) which cause 50% agglutination of 1 ml of 2% erythrocyte suspension at temperature of 25°C, and wherein said lectin preparation:
- is confectioned in the containers with marked level to which water can be introduced to obtain the suspension with hemaglutinic activity either 320HU or 640 HU/ml or
- is in a dry form of tablets containing dried lectin powder and talc and/or monosaccharides, preferably lactose and/or crystalline cellulose and/or lubricants, preferably magnesium stearate, and said tablets are coated with the film or sugar to facilitate swallowing and the hemaglutinic activity of each tablet or coated tablet or dragee is either 320HU or 640 HU or
- is in a dry form of gelatinate or cellulose capsules containing the dried lectin powder and the filler, wherein the hemaglutinic activity of each capsule is either 320HU or 640 HU.

2. A lectin preparation for use in increase in the weight gain in weaned piglet, where said lectin preparation is orally administered to said piglet one time between the 10th and 15th day of their life in dose comprising 320 haemagglutinic activity units (HU) for animals weighting below 2 kg and 640 HU for animals weighting 2-5 kg, wherein one unit of haemagglutinic activity (HU) is the quantity of material (in mg) which cause 50% agglutination of 1 ml of 2% erythrocyte suspension at temperature of 25°C, wherein said lectin preparation:
- is confectioned in the containers with marked level to which water can be introduced to obtain the suspension with hemaglutinic activity either 320HU or 640 HU/ml or
- is in a dry form of tablets containing dried lectin powder and talc and/or monosaccharides, preferably lactose and/or crystalline cellulose and/or lubricants, preferably magnesium stearate, and said tablets are coated with the film or sugar to facilitate swallowing and the hemaglutinic activity of each tablet or coated tablet or dragee is either 320HU or 640 HU or
- is in a dry form of gelatinate or cellulose capsules containing the dried lectin powder and the filler, wherein the hemaglutinic activity of each capsule is either 320HU or 640 HU.

## Patentansprüche

1. Verwendung von Lektin in der Herstellung emer Zubereitung zur Erhöhung der Gewichtszunahme im abgesetzten Ferkel, wobei die genannte Lektinzubereitung in trockener oder flüssiger Form ist und dem genannten Ferkel einmal zwischen ihrem zehnten und ftmfzehnten Lebenstag als eine Einheitsdosierung umfassend 320 hämagglutinische Aktivitätseinheiten (HU) für Tiere die unter 2 kg wiegen, und 640 HU für Tiere die 2 - 5 kg wiegen, oral verabreicht wird, wobei eine Einheit der hämagglutinischen Aktivität (HU) die Menge des Materials (in mg) ist, die 50% Agglutination von 1 ml einer 2 % Erythrozytensuspension bei einer Temperatur von 25°C verursacht, und worin genannte Lektinzubereitung:
- in Behältern mit markiertem Level bis wohin Wasser eingeführt werden kann, um die Suspension mit einer häfunagglutinischen Aktivität von entweder 320HU oder 640HU zu erhalten, konfektioniert ist, oder
- in einer trockenen Tablettenform ist, enthaltend getrocknetes Lektinpulver und Talk und/oder Monosaccharide, vorzugsweise Laktose und/oder kristalline Zellulose, und/oder Gleitmittel, vorzugsweise Magnesiumstearat, und wobei genannte Tabletten mit einem Film oder Zucker beschichtet sind, um das Schlucken zu erleichtern, und die hamagglutinische Aktivität jeder Tablette oder beschichteten Tablette oder Dragee entweder 320HU oder 640HU ist, oder
- in einer trockenen Form einer gelatinierten oder Zellulosekapsel ist, enthaltend das getrocknete Lektinpulver und der Füllstoff, wobei die hämagglutinische Aktivität jeder Kapsel entweder 320HU oder 640HU ist.

2. Eine Lektinzubereitung zur Erhöhung der Gewichtszunahme im abgesetzten Ferkel, wobei die genannte Lektinzubereitung dem genannten Ferkel einmal zwischen ihrem zehnten und fünfzehnten Lebenstag in einer Dosis umfassend 320 hämagglutinische Aktivitätseinheiten (HU) für Tiere die unter 2 kg wiegen, und 640 HU für Tiere die 2 - 5 kg wiegen, oral verabreicht wird, wobei eine Einheit der hämagglutinlschen Aktivität (HU) die Menge des Materials (in mg) ist, die 50% Agglutination von 1 ml einer 2 % Erythrozytensuspension bei einer Temperatur von 25°C verursacht, und worin genannte Lektinzubereitung:
- in Behältern mit markiertem Level bis wohin Wasser eingefuhrt werden kann, um die Suspension mit einer hämagglutinischen Aktivität von entweder 320HU oder 640HU zu erhalten, konfektioniert ist, oder :
- in einer trockenen Tablettenform ist, enthaltend getrocknetes Lektinpulver und Talk und/oder Monosaccharide, vorzugsweise Laktose und/oder kristalline Zellulose und/oder Gleitmittel, vorzugsweise Magnesiumstearat, und wobei genannte Tabletten mit einem Film oder Zucker beschichtet sind, um das Schlucken zu erleichtern, und die hämagglutinische Aktivität jeder Tablette oder beschichteten Tablette oder Dragee entweder 320HU oder 640HU ist, oder
- in einer trockenen Form einer gelatinierten oder Zellulosekapsel ist, enthaltend das getrocknete Lektinpulver und der Füllstoff, wobei die hämagglutinische Aktivität jeder Kapsel entweder 320HU oder 640HU ist,

## Revendications

1. Utilisation de lectine dans la fabrication d'une préparation visant à augmenter le gain de poids chez les porcelets sevrés, où ladite préparation de lectine est sous une forme sèche ou liquide et elle est administrée par voie orale auxdits porcelets une fois entre le 10^{e} et le 15^{e} jour de leur vie sous forme d'un dosage unitaire comprenant 320 unités d'activité hémagglutinique (HU) pour des animaux pesant moins de 2 kg et 640 HU pour des animaux pesant de 2 à 5 kg, où une unité d'activité hémagglutinique (HU) est la quantité de matériau (en mg) qui provoque 50 % d'agglutination de 1 ml d'une suspension érythrocytaire à 2 % à une température de 25 °C, et où ladite préparation de lectine :
- est confectionnée dans les récipients avec un niveau marqué jusqu'auquel de l'eau peut être introduite pour obtenir la suspension avec une activité hémaglutinique soit de 320 HU soit de 640 HU/ml ou
- est sous une forme sèche de comprimés contenant de la poudre de lectine séchée et du talc et/ou des monosaccharides, de préférence du lactose et/ou de la cellulose cristalline et/ou des lubrifiants, de préférence du stéarate de magnésium, et lesdits comprimés sont enrobés avec du film ou du sucre pour faciliter l'absorption et l'activité hémaglutinique de chaque comprimé ou comprimé enrobé ou dragée est soit de 320 HU soit de 640 HU ou
- est sous une forme sèche de capsules de gélatine ou de cellulose contenant la poudre de lectine séchée et l'agent de remplissage, où l'activité hémaglutinique de chaque capsule est soit de 320 HU soit de 640 HU.

2. Préparation de lectine pour une utilisation dans l'augmentation du gain de poids chez des porcelets sevrés, où ladite préparation de lectine est administrée par voie orale auxdits porcelets une fois entre le 10^{e} et le 15^{e} jour de leur vie dans une dose comprenant 320 unités d'activité hémaglutinique (HU) pour des animaux pesant moins de 2 kg et 640 HU pour des animaux pesant de 2 à 5 kg, où une activité hémaglutinique (HU) est la quantité de matériau (en mg) qui provoque 50 % d'agglutination de 1 ml d'une suspension érythrocytaire à 2 % à une température de ) 25 °C, où ladite préparation de lectine :
- est confectionnée dans les récipients avec un niveau marqué jusqu'auquel de l'eau peut être introduite pour obtenir la suspension avec une activité hémagglutinique soit de 320 HU soit de 640 HU/ml ou
- est sous une forme sèche de comprimés contenant de la poudre de lectine séchée et du talc et/ou des monosaccharides, de préférence du lactose et/ou de la cellulose cristalline et/ou des lubrifiants, de préférence du stéarate de magnésium, et lesdits comprimés sont enrobés avec du film ou du sucre pour faciliter l'absorption et l'activité unique de chaque comprimé ou comprimé enrobé ou dragée est soit de 320 HU soit de 640 HU ou
- est sous une forme sèche de capsules de gélatine ou de cellulose contenant la poudre de lectine séchée et l'agent de remplissage, où l'activité hémaglutinique de chaque capsule est soit de 320 HU soit de 640 HU.
